# DEMANDE DE BREVET EUROPEEN

(11) **EP 0 625 333 A1**
(43) Date de publication de la demande: **23.11.1994**
(21) Numéro de dépôt: 93107751.5
(22) Date de dépôt: 12.05.1993
(51) Int. Cl.: A61B 5/14

(54) **Procédé et dispositif pour le prélèvement d'échantillon de liquide corporel**

(71) Demandeur: HOSPAL INDUSTRIE, F-69883 Meyzieu Cédex (FR); COBE LABORATORIES, INC., Lakewood CO 80215-4407 (US)
(72) Inventeur: Urdahl, Steven G., Golden, Colorado, 80401 (US); Lejeune, Daniel, F-60560 Orry-la-Ville (FR)
(74) Mandataire: Lejeune, Daniel

(57) **Abrégé**

Un dispositif de prise d'échantillon de liquide corporel (84) dans un tube (72) muni de moyens de prise d'échantillon (88), le tube (72) étant sensiblement compressible sur au moins une partie de sa longueur et ayant une première extrémité connectable à un conteneur (64) pour un liquide médical (68), comprend des moyens de compression (96) montés sur le tube (72) pour coopérer avec le tube (72) dans une première position où le passage est libre à l'intérieur du tube (72) et dans une seconde position où le tube (72) est obturé par un point de compression, les moyens de compression (96) pouvant être déplacés par glissement le long du tube (72) dans la seconde position, et le glissement des moyens de compression (96) provoquant un déplacement de liquide dans le tube (72) en direction et/ou à partir du conteneur (64).

Ce dispositif est particuliérement adapté à la prise d'échantillon de sang à partir d'une ligne de perfusion.

## Description

L'invention concerne un procédé et un dispositif pour le transfert de liquide médical ou corporel et, plus précisément, un procédé et un dispositif pour déplacer un liquide médical ou corporel dans un tube compressible. L'invention trouve une application particulière dans les systèmes de séparation des composants du sang (par exemple, plaquettes) et dans les systèmes combinés de perfusion de liquide médical et de prise d'échantillon de sang.

Des assemblages formés d'un conteneur connecté à un tube ont de nombreuses applications médicales. Une utilisation connue en est faite dans les systèmes combinés de perfusion de liquide médical et de prise d'échantillon de sang. Dans cette application, un liquide, tel qu'une solution saine contenue dans un conteneur, est perfusée à un patient par un tube auquel est connectée une canule. Des moyens de prise d'échantillon de sang (par exemple, un site d'accès en ligne) sont disposés entre le conteneur et la canule, et des moyens stationnaires pour arrêter l'écoulement du liquide sont disposés entre les moyens de prise d'échantillon de sang et le conteneur. Des moyens de purge en ligne sont souvent connectés au tube entre les moyens de prise d'échantillon de sang et les moyens pour arrêter l'écoulement de liquide. Les moyens de purge en ligne peuvent comporter par exemple un second site d'accès en ligne et une seringue qui peut y être connectée et déconnectée à volonté, ou un réceptacle préconnecté au tube pour recevoir une partie de la solution contenue dans le tube avant la prise d'échantillon de sang.

Pour préparer la prise d'échantillon de sang proprement dite, les moyens pour arrêter l'écoulement de liquide sont disposés en position d'obturation et au moins une partie de la solution de perfusion se trouvant en aval dans le tube est transférée dans les moyens de purge en ligne (par exemple, par aspiration de liquide dans une seringue par l'intermédiaire du site d'accès en ligne). Simultanément, du sang est admis dans le tube par l'intermédiaire de la canule, laquelle est donc connectée à une portion du tube située en aval des moyens de purge en ligne. Un échantillon de sang est alors prélevé dans le tube pour analyse par l'intermédiaire des moyens de prise d'échantillon de sang (par exemple, au moyen d'une seringue connectée au site d'accès).

Les systèmes décrits ci-dessus comportent un certain nombre d'inconvénients. Par exemple, dans le système comprenant plusieurs sites d'accès en ligne, deux seringues sont utilisées et sont jetées après chaque prise d'échantillon de sang (voir par exemple le brevet US 4 763 648). Cela représente une dépense non négligeable, et pour les seringues elles-mêmes et pour leur mise au rebut, et cela entraîne un temps de main d'oeuvre significatif de la part du personnel chargé de la manipulation des seringues. Mais surtout l'utilisation de ces systèmes emporte les risques de contamination par le sang, de rupture de stérilité du système de perfusion, et de piqûre par les aiguilles. Le besoin d'utiliser deux seringues à chaque prise de sang est évité dans les systèmes qui comprennent un réceptacle de purge connecté en ligne (voir par exemple les brevets US 4 673 386 et 5 002 066). De tels systèmes sont cependant souvent au moins aussi coûteux que ceux qui ont été mentionnés plus haut dans la mesure où ils nécessitent la préconnexion du réceptacle de purge en ligne, et où l'ensemble des composants de ces systèmes doit être stérilisé avant l'utilisation et être jeté après une utilisation unique. En outre un système ayant un réceptacle de purge préconnecté en ligne est aussi plus volumineux et nécessite pour son utilisation des moyens de support spécifiques.

Une autre utilisation médicale d'un assemblage formé d'un tube connecté à un conteneur est faite dans les systèmes de séparation des composants du sang. Dans de tels systèmes, du sang est prélevé d'un patient ou donneur et est introduit dans une centrifugeuse où les composants du sang sont séparés par centrifugation. Par exemple, un fluide corporel contenant des plaquettes peut être isolé et collecté par un tube reliant la centrifugeuse à un conteneur. Lorsque le processus de centrifugation est achevé, il est souvent nécessaire de déterminer la concentration en plaquettes du liquide médical recueilli. Pour ce faire, on prélève usuellement une portion du tube contenant le liquide et on en analyse le contenu. Pour être sûr d'obtenir un échantillon représentatif du contenu du conteneur, on utilise habituellement un outil tenu à la main (du type d'une pince ayant deux mors cylindriques disposés en vis-à-vis pour comprimer le tube) pour évacuer dans le conteneur le liquide contenu dans le tube. Ensuite on provoque une turbulence dans le conteneur, par exemple en le remuant, pour en homogénéiser le contenu. Enfin, on laisse le liquide contenant les plaquettes remplir à nouveau le tube dont on prélève ultérieurement une portion. Comme on le conçoit aisément, l'utilisation de la pince pour comprimer le tube dans ce processus nécessite à la fois de serrer la pince et de la déplacer en direction du conteneur. Par ailleurs il faut la maintenir serrée lorsque l'on mélange le contenu du conteneur, ce qui ne rend pas la manipulation aisée. Enfin, il faut avoir une telle pince à sa disposition et la maintenir en état (désinfection, etc).

L'invention a pour objet un nouveau procédé et un nouveau dispositif pour déplacer des liquides médicaux ou corporels dans un système de transfert. L'invention présente des avantages significatifs par rapport à l'art antérieur, en ce qu'elle permet d'obturer un tube sensiblement compressible sur au moins une partie de sa longueur par des moyens de compression qui maintiennent l'occlusion sans nécessiter une action continue de la part de l'utilisateur, de déplacer le liquide dans le tube en faisant glisser les moyens de compression le long du tube, et de maintenir le tube obturé par les moyens de compression sans l'intervention de l'utilisateur, l'utilisateur ayant alors les mains libres pour d'autres manipulations. De préférence, les moyens de compression sont configurés de façon que, une fois montés sur le tube, celui-ci puisse être obturé àvolonté, et qu'ils puissent être déplacés par glissement par rapport au tube, qu'ils soient disposés pour obturer le tube, ou qu'ils soient disposés pour laisser le passage libre dans le tube. En outre, des moyens de préhension peuvent être connectés au tube pour permettre de saisir le tube d'une main, tandis que l'autre main déplace les moyens de compression dans une direction opposée aux moyens de préhension. Comme on le comprendra, dans certaines applications il est en outre souhaitable que les moyens de préhension remplissent leurs fonctions sans qu'une compression sensible du tube en résulte.

Dans l'un des modes de réalisation de l'invention, un assemblage est prévu pour une utilisation dans un système combiné de perfusion de liquide médical et de prise d'échantillon de sang. Cet assemblage comprend un conteneur de liquide médical, un tube sensiblement compressible sur au moins une partie de sa longueur ayant une extrémité connectable au conteneur et une autre extrémité connectable à une canule, des moyens de prise d'échantillon de sang en ligne, et des moyens de compression disposés sur le tube entre les moyens de prise d'échantillon de sang et le conteneur de liquide médical. Pour faciliter la fabrication et la mise en oeuvre, les moyens de compression peuvent comprendre une plaque de plastique moulée ayant une ouverture en trou de serrure, destinée au passage du tube. Une extrémité de l'ouverture est dimensionnée pour accommoder le tube sans l'obturer, tandis que l'autre extrémité de l'ouverture est dimensionnée pour accommoder le tube de façon à l'obturer par compression. Les moyens de prise d'échantillon de sang peuvent comprendre un site d'accès en ligne classique, peu coûteux. Bien que les moyens de prise d'échantillon de sang puissent être utilisés comme moyens de préhension, un collier cylindrique peut aussi être assujetti au tube à cette fin, et il est alors choisi suffisamment rigide pour prévenir toute compression sensible du tube lorsqu'il est utilisé. Dans ce mode de réalisation, l'invention utilise avantageusement le conteneur de liquide médical non seulement pour contenir le liquide médical lors de la perfusion du liquide au patient, mais aussi pour recueillir le liquide médical provenant du tube lors de la prise d'échantillon de sang.

Dans une phase préparatoire à la prise d'échantillon de sang, les moyens de compression sont disposés de façon à obturer le tube par compression (par exemple, en engageant le tube à force dans la partie de l'ouverture ayant une largeur réduite). Un des intérêts du dispositif est que l'occlusion se maintient sans que l'utilisateur ait à intervenir plus avant. On fait alors glisser les moyens de compression le long du tube de façon à déplacer le point de compression. Ce déplacement du point de compression sur le tube a pour effet non seulement de transférer le liquide médical du tube dans le conteneur, mais aussi de provoquer la remontée du sang du patient dans le tube en aval des moyens de compression (sous l'effet d'un phénomène d'aspiration et, éventuellement de la pression sanguine du patient). On notera que le mot "aval" tel qu'il est utilisé ici s'applique à des emplacements ou à des composants qui sont situés du côté des moyens de compression opposé au conteneur, indépendamment de la direction de l'écoulement dans le tube à un instant particulier. Lorsque l'utilisateur a fait glisser les moyens de compression sur le tube de la façon décrite ci-dessus jusqu'à un point suffisamment éloigné sur le tube pour permettre le prélèvement d'un échantillon de sang non dilué à partir des moyens de prise d'échantillon, il peut lâcher les moyens de compression et, ayant alors les deux mains libres, il peut prélever un échantillon de sang par les moyens de prise d'échantillon de sang (par exemple, en connectant une seringue dans un site d'accès en ligne). Ensuite, le passage peut être rétabli dans le tube par désengagement des moyens de compression, le tube étant poussé dans la partie de l'ouverture ayant la plus grande largeur.

Bien que les moyens de compression puissent occuper toute une gamme de positions le long du tube préalablement à leur engagement occlusif et à leur glissement le long du tube comme cela a été décrit plus haut, il faut, pour qu'un échantillon de sang non dilué puisse être prélevé par les moyens de prise d'échantillon, qu'ils soient positionnés initialement de telle sorte que la distance qui les sépare du conteneur soit plus grande que la distance qui les sépare du patient. Par conséquent, avant d'arrêter le déplacement occlusif des moyens de compression sur le tube, le liquide dans le tube doit être déplacé sur une distance suffisante pour que le tube ne soit rempli que de sang de part et d'autre des moyens de prise d'échantillon de sang. De cette façon, un échantillon de sang non dilué, propre à l'analyse, peut être obtenu en une seule prise d'échantillon, contrairement à ce qui peut être réalisé avec beaucoup de systèmes existants dans lesquels le premier échantillon prélevé est un mélange de liquide médical et de sang qui doit être mis au rebut avant qu'un échantillon de sang non dilué puisse être prélevé.

Compte tenu de ce qui précède, on comprendra que ce mode de réalisation de l'invention présente de nombreux avantages. Par exemple, les coûts de fabrication sont réduits puisque le seul composant en ligne requis est un organe de prise d'échantillon de sang. Par comparaison, la plupart des systèmes actuels comportent des moyens de prise d'échantillon en ligne et des moyens de purge en ligne pour le transfert de liquide (solution de perfusion, et en général, sang) du tube dans les moyens de purge jusqu'à ce qu'il soit possible de prélever un échantillon de sang non dilué par les moyens de prise d'échantillon. Avec le dispositif selon l'invention, il n'y a pas de perte de sang, puisque le liquide médical dans le tube est transféré dans le conteneur jusqu'à ce qu'un échantillon de sang non dilué puisse être prélevé par les moyens de prise d'échantillon. Dans beaucoup de systèmes existants, avant qu'un échantillon de sang non dilué puisse être prélevé, il faut d'abord prélever un échantillon de liquide médical et de sang mélangés, lequel doit être mis au rebut, ce qui a aussi pour conséquence d'augmenter les coûts en matériel. En outre, les étapes nécessaires pour prélever un échantillon de sang propre à l'analyse selon l'invention demandent un temps moindre et présentent beaucoup moins de risques pour l'utilisateur que la manipulation de certains systèmes actuels (l'usage des seringues est réduit).

Dans un autre mode de réalisation de l'invention, un assemblage est prévu pour être utilisé avec des systèmes de séparation des composants du sang. Cet assemblage comprend un conteneur pour recevoir un composant déterminé d'un fluide corporel, tel que du plasma contenant des plaquettes, un tube connecté àune extrémité au conteneur et connectable à son autre extrémité, par exemple, àune centrifugeuse, et des moyens de compression tels que décrits plus haut, qui sont montés sur le tube, lequel est sensiblement compressible sur au moins une partie de sa longueur. En outre, des moyens de préhension peuvent être avantageusement connectés au tube entre les moyens de compression et la centrifugeuse.

Lors de la préparation pour les opérations de séparation des composants du sang, un volume de sang est transféré dans la centrifugeuse. Pour ce faire, la centrifugeuse peut être directement connectée à un patient ou un donneur pour un processus continu dans lequel le sang du patient ou donneur est dérivé directement dans la centrifugeuse et lui est restitué, après centrifugation, dépourvu du ou des composants à collecter.

Le composant souhaité est séparé du reste du sang dans la centrifugeuse et il est ensuite transféré dans un conteneur de recueil. Lorsque la centrifugation est terminée, le tube est scellé. Les moyens de compression sont alors disposés sur le tube de façon à l'obturer. De préférence, les moyens de compression sont disposés en un point aussi proche que possible du lieu ou le tube est scellé de façon que la quantité de liquide transférée du tube dans le conteneur soit la plus importante possible. A cet effet, les moyens de compression sont déplacés par glissement le long du tube dans la direction du conteneur pour produire le déplacement d'un point de compression le long du tube. Ce glissement du point de compression sur le tube déplace le liquide dans le tube entre les moyens de compression et le conteneur, et a pour résultat le transfert du liquide contenu dans le tube dans le conteneur. De préférence, les moyens de compression sont déplacés jusqu'à un point aussi proche que possible du conteneur pour que la quantité de liquide transférée du tube dans le conteneur soit la plus grande possible. L'utilisateur peut alors lâcher les moyens de compression et avoir l'usage de ses deux mains pour homogénéiser mécaniquement le liquide à l'intérieur du conteneur, par exemple en faisant osciller le conteneur. Après que le liquide a été mélangé de façon adéquate dans le conteneur, l'utilisateur manipule les moyens de compression pour rétablir le passage à l'intérieur du tube. Il en résulte que du fluide s'écoule dans le tube de sorte qu'un échantillon, désormais représentatif de la concentration du composant désiré dans le liquide, peut alors être prélevé.

Compte tenu de ce qui précède, on comprendra que le mode de réalisation qui vient d'être décrit présente plusieurs avantages par rapport aux systèmes de séparation des composants du sang actuels. Par exemple, l'élément utilisé pour déplacer le liquide, c'est-à-dire les moyens de compression, est bon marché et facile à utiliser. En outre, une fois que les moyens de compression sont disposés sur le tube de façon à l'obturer, aucune force extérieure additionnelle n'est requise pour maintenir l'occlusion. Cela est avantageux non seulement parce que la force requise de l'utilisateur pour faire glisser les moyens de compression en direction du conteneur est réduite, mais aussi parce que l'utilisateur peut lâcher les moyens de compression à tout moment au cours du processus. En revanche, la pince utilisée actuellement pour obturer le tube nécessite que l'utilisateur maintienne son effort pour que le tube reste obturé, même après que la pince est positionnée à proximité du conteneur. L'utilisateur doit donc garder la pince serrée sur le tube lorsqu'il mélange le contenu du conteneur, ce qui rend cette manipulation malaisée. Outre leur facilité d'utilisation, les moyens de compression selon l'invention présentent aussi l'avantage d'être aisément disponibles et de ne pas constituer source potentielle de contamination, étant à usage unique.

D'autres caractéristiques et avantages de l'invention apparaîtront à la lecture de la description qui va suivre. On se reportera aux dessins annexés sur lesquels :
la Figure 1 représente les éléments de l'invention ;
les Figure 2 à 6 représentent un mode de réalisation de la présente invention dans un système combiné de perfusion de liquide et de prise d'échantillon de sang ; et
les Figures 7 à 11 représentent un autre mode de réalisation d'invention dans un système de séparation des composants du sang.

D'une façon générale, l'invention est un procédé et un dispositif de transfert d'un liquide médical ou corporel dans lequel le déplacement d'un point de compression, produit par des moyens de compression, le long d'un tube sensiblement compressible, est utilisé pour déplacer du liquide à des fins diverses, telles que pour permettre la prise d'un échantillon de liquide corporel à partir d'un tube de perfusion ou pour améliorer l'homogénéité d'un échantillon d'un composant d'un liquide corporel séparé par un procédé connu (par exemple, centrifugation).

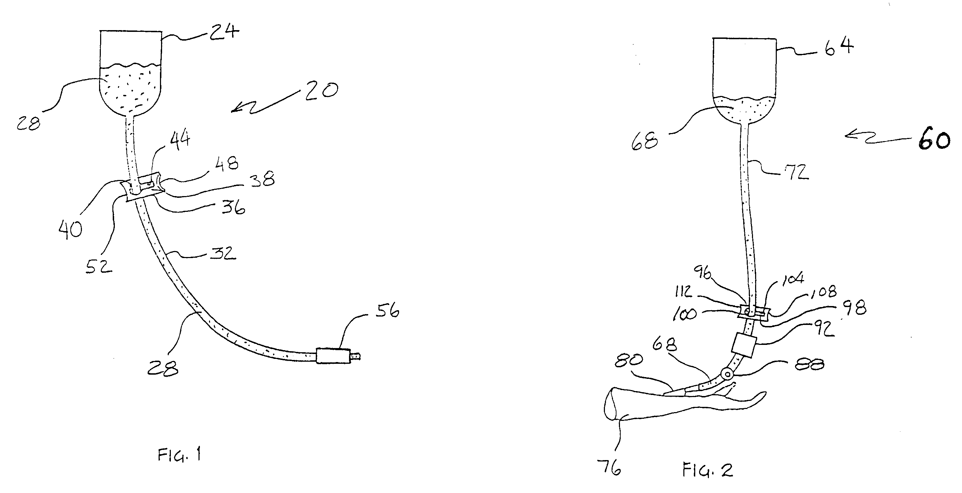

L'invention est illustrée dans son principe sur la Figure 1. Le système de transfert de liquide médical ou corporel représenté comprend un conteneur 24 pour liquide médical ou corporel 28, un tube 32 constituant une voie d'écoulement pour le liquide 28 en direction de, et à partir du conteneur 24, et des moyens de compression péristaltiques, tels qu'un clamp mobile 36, pour produire sur le tube un point de compression glissant et déplacer le liquide 28 à l'intérieur du tube 32 en direction de ou à partir du conteneur 24. Bien que l'utilisateur puisse tenir directement le tube 32 durant ce glissement forcé du clamp 36 (pour engendrer les forces nécessaires au déplacement du clamp, ou pour minimiser les effets des forces engendrées sur des connexions disposées en aval du tube, comme au patient), un point de préhension 56 peut également assujetti au tube 32 pour fournir une prise à l'utilisateur durant cette étape du processus. Dans les cas où il est souhaitable de ne pas entraver le déplacement du liquide dans le tube 32 en aval du clamp 36 (comme cela a été mentionné plus haut "en aval" s'applique à la portion du tube 32 située à l'opposé du conteneur par rapport au clamp 36), tel que dans le procédé de prise d'échantillon de sang qui a été mentionné plus haut, le point de préhension 56 peut être fait d'un matériau sensiblement incompressible permettant à l'utilisateur de saisir fermement le tube 32 sans causer son occlusion.

Afin que le clamp 36 puisse produire un point de compression effectif sur le tube 24, il comprend une plaque munie d'une ouverture 38 en trou de serrure ayant une première et une seconde extrémité 40, 44. La première extrémité 40 de l'ouverture est dimensionnée de telle sorte que lorsque le tube 32 s'y trouve logé, il ne se produit pas d'obturation sensible du tube 32 (c'est-à-dire qu'aucune compression sinon insignifiante n'y est appliquée). Cela permet de laisser le clamp 36 sur le tube 32 quand il est souhaitable ou nécessaire qu'un liquide s'y écoule, à partir de ou en direction du conteneur 24, et cela permet aussi de positionner le clamp 36 à l'emplacement désiré sur le tube sans que l'écoulement du liquide n'en soit affecté. Quand il est nécessaire de déplacer le liquide 28 dans le tube 32 en direction du conteneur 24, le tube 32 est introduit dans la seconde extrémité 44 de l'ouverture 38, laquelle est dimensionnée pour produire une occlusion du tube 32 par compression. Une fois que le tube 32 est disposé dans la seconde extrémité 44, aucune force additionnelle n'a besoin d'être appliquée sur le clamp 36 pour maintenir l'occlusion du tube (c'est-à-dire que l'occlusion résulte de la seule interaction entre le clamp 36 et le tube 32). Pour obtenir les résultats mentionnés plus haut, dans un des modes de réalisation dans lequel le tube 32 est formé de PVC (chlorure de polyvinyle) flexible et a un diamètre interne d'environ 0,287 cm (0,113 pouce) et une épaisseur de paroi d'environ 0,094 cm (0,037 pouce), le clamp 36 est fait de HDPE (polyéthylène haute densité), la première extrémité 40 de l'ouverture 38 est sensiblement circulaire et a un diamètre d'environ 0,76 cm (0,30 pouce) et la partie obturante 44 de l'ouverture 38 est une fente qui s'étend sur environ 1,27 cm (0,50 pouce) et a une largeur augmentant régulièrement de environ 0,089 cm (0,035 pouce) à environ 0,068 cm (0,027 pouce).

Pour provoquer l'occusion du tube 38 par le clamp 36, l'utilisateur (non représenté) saisit le clamp 36, de préférence par les première et seconde surfaces d'engagement 48 et 52 qui sont concaves pour faciliter la préhension, et il introduit le tube 32 dans la seconde extrémité 44 de l'ouverture 38. Une fois que le clamp 36 est engagé sur le tube 32 de cette façon, l'utilisateur saisit le clamp 36 d'une main et le tube 32 de l'autre main et fait glisser le clamp 36 en direction du conteneur 24 le long du tube 32 pour provoquer le déplacement du point de compression, c'est-à-dire aussi le déplacement du liquide dans le tube 32 entre le clamp 36 et le conteneur 24 et le transfert du liquide dans le conteneur 24. Dans les cas où il est souhaitable que le liquide puisse s'écouler librement dans le tube 32 en aval du clamp 36 au cours de ce mouvement (par exemple le sang, lors de la prise d'un échantillon de sang), l'utilisateur saisit le point de préhension 56 de sorte que l'écoulement de liquide n'est pas entravé.

Afin de faciliter le déplacement du point de compression sur le tube 32, les parties du clamp 36 qui viennent en contact avec le tube 32 peuvent être faites d'un matériau ayant un coefficient de friction réduit, bien que dans le mode de réalisation préféré le clamp 36 soit fait d'un seul matériau. Dans un mode de réalisation où le tube 32 est fait de PVC flexible et le clamp 36 est fait de HDPE, comme cela a été décrit plus haut, l'interaction entre les composants produit la friction réduite souhaitée.

Bien que les moyens de compression péristaltiques décrits plus haut l'aient été en référence à une structure particulière (clamp 36), les spécialistes du domaine comprendront que différents types de structure peuvent offrir des caractéristiques fonctionnelles similaires ou équivalentes, à savoir permettre une occlusion sélective du tube 32, permettre le déplacement d'un point de compression par glissement sur le tube 32, et supprimer la nécessité pour l'utilisateur d'exercer une force pour maintenir l'occlusion une fois que le tube est obturé. C'est ainsi qu'une pince qui pourrait être positionnée sur le tube 32 à volonté pour établir ce type d'interaction avec le tube 32 est comprise dans le champ de l'invention (par exemple, une pince ne nécessitant pas d'être mise en place sur le tube 32 par une extrémité libre du tube 32).

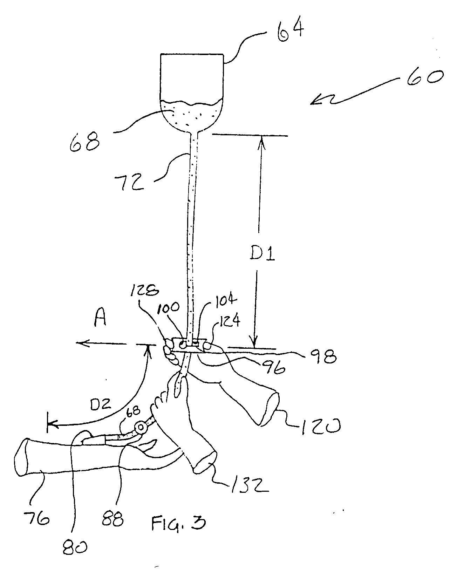

Les principes du système 20 de transfert de liquide médical ou corporel qui vient d'être décrit peuvent être utilisés dans des applications variées, incluant le système 60 de prise d'échantillon de sang représenté sur les Figures 2 à 6. Tel que représenté sur la Figure 2, ce système comprend un conteneur 64 pour un liquide médical 68 destiné à être perfusé à un patient par l'intermédiaire d'un tube 72. De façon plus détaillée, un connecteur intraveineux 80 est connecté à un bras 76 du patient et le tube 72 est assujetti au connecteur 80, de façon que le liquide médical 68 puisse s'écouler librement du conteneur dans le système vasculaire du patient. Afin de pouvoir prélever sur le patient des échantillons de sang 84, tandis que le patient est connecté au conteneur 64, un site d'accès 88 connu en soi (par exemple, du type permettant l'insertion de l'aiguille d'une seringue (non représentée) et qui se referme sur soi-même lorsque l'aiguille en est retirée) est disposé sur le tube 72, et un clamp 96 du type décrit plus haut est monté sur le tube 72 entre le site d'accès 88 et le conteneur 64. Comme cela a été noté plus haut à propos du système 20 de transfert de liquide médical ou corporel, le clamp 96 est utilisé pour obturer le tube 72 par compression et il peut être déplacé sur le tube en direction du conteneur 64 pour y transférer du liquide. Ce déplacement provoque aussi la remontée du sang 84 dans le tube 72 en aval du clamp 96 en direction du site d'accès 88. Bien que l'utilisateur puisse saisir l'un des composants du système 60 tel que le site d'accès 88 ou le tube 72 lui-même durant le mouvement de glissement forcé du clamp 96 (pour engendrer les forces nécessaires à ce déplacement et pour minimiser les effets de ces forces sur les connexions situées en aval sur le tube 72), il faut que l'écoulement du sang 84 en direction du site d'accès 88 n'en soit pas entravé. En vue d'éliminer la possibilité d'une obturation indésirable du tube 72, un collier 92 fait d'un matériau sensiblement incompressible peut être assujetti au tube 72 afin de fournir un point de préhension à une main de l'utilisateur tandis que, de son autre main, il déplace le clamp 96.

Pendant la perfusion du liquide médical 68 au patient, le clamp 96 est généralement dans la position représentée sur la Figure 2, c'est-à-dire que le tube 72 est placé dans la première extrémité 100 de l'ouverture 98 qui, comme mentionné plus haut, est dimensionnée de façon à ne pas obturer le tube 72. Le liquide médical 68 peut donc s'écouler librement dans le bras 76 du patient par le connecteur intraveineux 80. Lorsqu'il apparaît souhaitable de prélever un échantillon de sang 84, l'utilisateur saisit le clamp 96 comme représenté sur la Figure 3. De façon plus détaillée, l'utilisateur peut prendre le clamp 96, en particulier les première et seconde surfaces de préhension 108, 112, entre le pouce 124 et l'index 128 d'une main 120, et saisir le tube 72 de l'autre main 132 en toute position adéquate. L'utilisateur peut alors engager le champ 96 sur le tube 72 en faisant pénétrer à force le tube dans la seconde extrémité 104 de l'ouverture 98 afin de comprimer le tube 72 (direction de la flèche A, sur la Figure 3). Comme cela a été mentionné plus haut, la seconde extrémité 104 de l'ouverture 98 est dimensionnée de façon que le tube 72 soit sensiblement obturé lorsqu'il y est engagé (c'est-à-dire, de façon que les parois intérieures du tube 72 soient mises en contact par une compression forcée). Il en résulte que l'écoulement de liquide médical 68 à partir du conteneur 64 en direction du bras du patient 76 est interrompu, et, d'autre part, que toute remontée de sang en amont du champ 96 est rendue impossible quelles que soient les manipulations effectuées ultérieurement. Le contenu du conteneur 64 ne peut donc être pollué en aucun cas par du sang.

De façon avantageuse, une fois que le clamp 96 a été engagé dans cette position par l'utilisateur, il n'est plus nécessaire d'exercer aucune force sur le clamp 96 pour maintenir l'occlusion (c'est-à-dire que l'engagement occlusif est maintenu par la simple interaction entre le tube 72 et la seconde extrémité 104 du clamp 96, le clamp 96 étant autobloquant une fois qu'il est dans la position décrite).

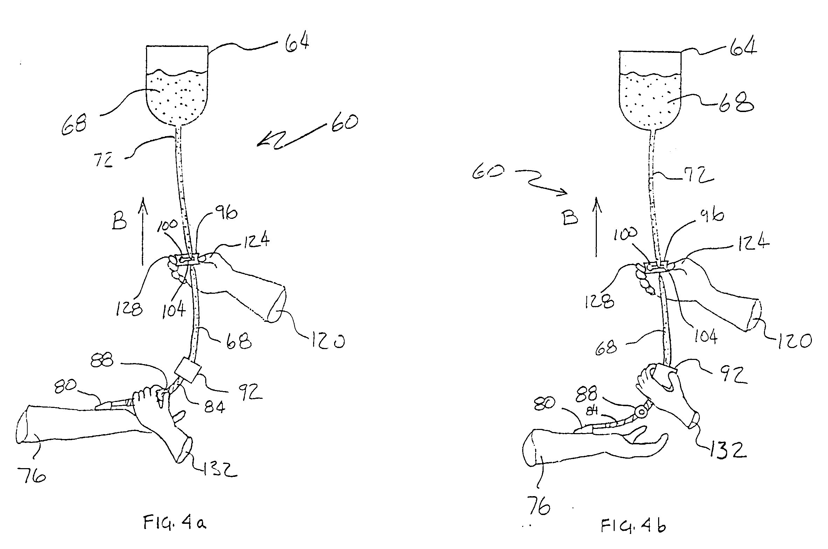

Une fois que le clamp 96 est placé en position d'occlusion sur le tube 72, l'utilisateur fait glisser le clamp 96 sur le tube 72 en direction du conteneur 64, c'est-à-dire dans la direction de la flèche B sur les Figure 4A et 4B. Ce déplacement provoque le déplacement du liquide médical 68 dans le tube 72 entre le clamp 96 et le conteneur 64, et le volume du liquide médical 68 dans le conteneur 64 en augmente d'autant. Afin d'aider l'utilisateur à fournir les forces nécessaires pour produire le déplacement par glissement du clamp 96, ainsi que pour réduire au maximum l'action de ces forces sur les interconnexions du tube 72 en aval du clamp 96, l'utilisateur peut saisir le site d'accès (Figure 4A) ou le collier 92 (Figure 4B) de son autre main 132. Outre le déplacement du liquide médical 68 entre le clamp 96 et le conteneur 64, le glissement du clamp 96 et le déplacement du point de compression qu'il engendre sur le tube 72 provoque aussi la remontée de sang 84 dans le tube 72 en aval du clamp 96 (par un phénomène d'aspiration résultant de la décompression du tube 72 consécutive au déplacement du point de compression sur celui-ci produit par le clamp 96, c'est-à-dire de l'expansion du tube 72 lorsqu'il n'est plus soumis à la compression du clamp 96, grâce aux propriétés élastiques du matériau dont est fait le tube et à la pression sanguine du patient). Comme l'objet du système 60 de prise d'échantillon est de prélever un échantillon de sang 84, il est souhaitable que l'écoulement du sang 84 dans le tube 72 ne soit pas affecté de manière significative lors de la préhension du système 60 par l'utilisateur durant le déplacement du clamp 96 qui vient d'être décrit. Plus spécifiquement, l'occlusion du tube 72 en aval du clamp 96 durant le déplacement du clamp doit donc être évitée. Bien qu'il soit possible pour l'utilisateur de saisir le site d'accès en ligne 88 ou le tube 72 lorsqu'il déplace le clamp 96 sans provoquer une telle occlusion, le tube 72 est de préférence muni du collier 92 décrit plus haut afin de fournir à l'autre main de l'utilisateur un point de saisie commode. Ce collier 92 est fait d'un matériau sensiblement incompressible afin de rendre impossible l'occusion du tube 72 lorsqu'il est saisi par l'utilisateur.

Lorsque le tube 72 est initialement obturé par manipulation du clamp 96, le tube 72 contient du liquide médical en aval du clamp 96. Pour que le sang 84 prélevé dans l'opération de prise d'échantillon qui va être décrite plus loin ne soit pas dilué, c'est-à-dire aussi pour éviter d'avoir à mettre au rebut une seringue remplie d'un mélange de sang 84 et de liquide médical 68 préalablement à la prise d'échantillon de sang proprement dit, le clamp 96 doit être déplacé le long du tube 72 de telle façon que du sang 84 non dilué soit aspiré au moins jusqu'au site d'accès 88 et, de préférence, jusqu'à un point situé entre le clamp 96 et le site d'accès 88. Ce résultat peut être obtenu par le positionnement occlusif initial du clamp 96 sur le tube 72 en un point tel que la distance (D1 sur la Figure 3) entre le clamp 96 et le conteneur 64 est égale ou de préférence supérieure à la distance (D2 sur la Figure 3) entre le clamp 96 et le bras du patient 76.

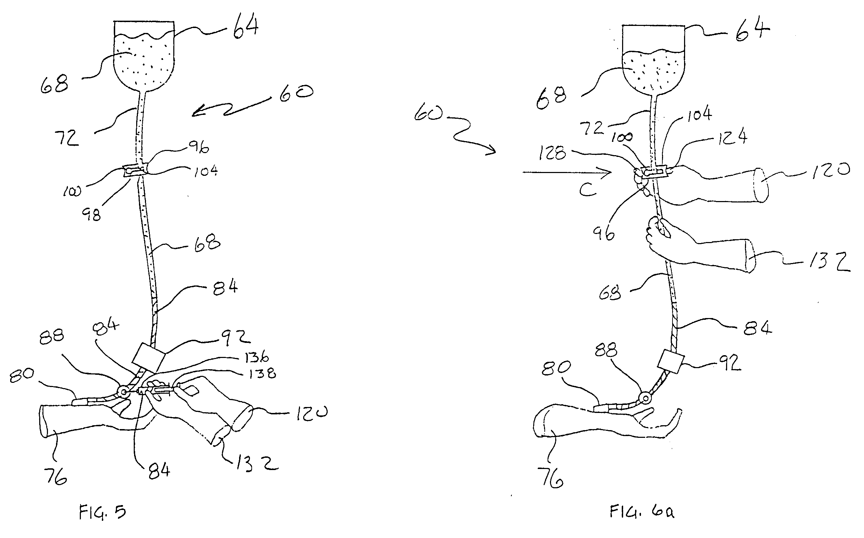

Lorsque le clamp 96 a été déplacé de la façon décrite plus haut jusqu'à la position illustrée sur la Figure 5, le sang 84 remplit le tube 72 au-delà du site d'accès 88, de sorte que seul du sang non dilué sera prélevé lors de la prise d'échantillon. Comme l'interaction entre le clamp 96 et le tube 72 produit d'elle-même les forces nécessaires pour maintenir l'occlusion du tube 72, l'utilisateur a les mains libres pour les manipulations ultérieures. De la sorte, il peut maintenir le site d'accès 88 d'une main, tandis qu'il tient une seringue 136 de l'autre main 120. Le prélèvement de sang proprement dit est effectué en enfonçant l'aiguille de la seringue 136 dans le site d'accès 88 et en tirant le piston le piston 138 vers l'arrière pour aspirer du sang 84 dans la seringue tel qu'illustré sur la Figure 5.

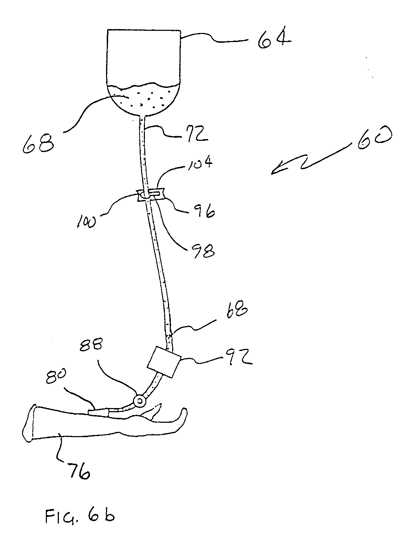

Une fois que l'échantillon de sang 84 a été prélevé de la façon décrite, l'utilisateur peut manipuler le clamp 96 pour rétablir l'écoulement de liquide médical dans le tube 72. De façon plus spécifique, l'utilisateur peut prendre les première et seconde surfaces de préhension 108, 112 du clamp 96 entre le pouce 124 et l'index 128 d'une main 124 afin de déplacer le clamp 96 dans la direction de la flèche C sur la Figure 6A, de sorte que le tube 72 soit à nouveau placé dans la première extrémité 100 de l'ouverture 98, comme cela est représenté sur le Figure 6B. Comme la première extrémité 100 est dimensionnée de façon à ne pas obturer le tube 72, le liquide médical 68 s'écoule à nouveau dans le bras du patient 76 par le tube 72 et le connecteur intraveineux 80. Une fois encore, pour se faciliter la manipulation du clamp 96, l'utilisateur peut saisir ou le site d'accès 88, ou le collier 92, ou même le tube 72, puisqu'une obturation temporaire du tube 72 à ce stade du processus est sans conséquence.

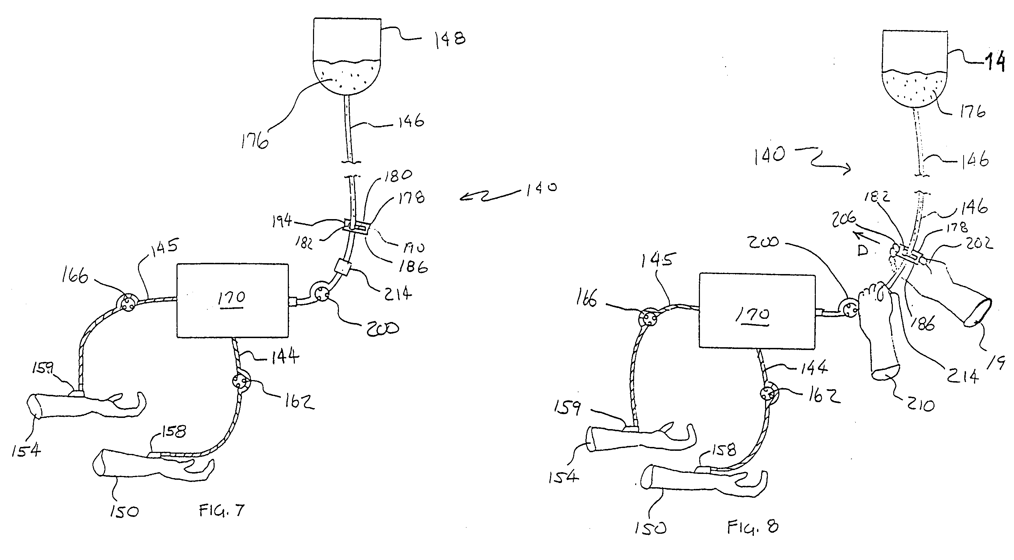

Une autre application utilisant les principes du système 20 de transfert de liquide médical ou corporel de la Figure 2 est le système 140 de séparation de composants du sang représenté sur les Figure 7 à 11. Le système de séparation 140 comprend une centrifugeuse 170 qui est connectée à une source de liquide corporel et à un conteneur 148 pour le recueil du composant récolté. Dans un mode de réalisation, la séparation peut se faire selon un processus continu en connectant le patient à la centrifugeuse 170. Plus spécifiquement, un bras 150 du patient est connecté à la centrifugeuse 70 par un connecteur intraveineux 158 et un tube compressible 144 pour acheminer le sang du patient dans la centrifugeuse, tandis qu'un autre tube compressible 145, muni à son extrémité libre d'un connecteur intraveineux 159 connecté à l'autre bras 154, est utilisé pour restituer au patient le sang dépourvu du composant récolté. Une pompe amont 162 et une pompe aval 166 disposées respectivement sur les tubes 144, 145 sont utilisées pour faire circuler le liquide corporel. La centrifugeuse 170 est aussi connectée à un conteneur de recueil 148 dans lequel est récolté le composant du sang souhaité, séparé au moyen de la centrifugeuse 170. Comme cela est connu, des composants particuliers d'un liquide donné peuvent être séparés dans une centrifugeuse 170 grâce aux forces centrifuges produites par la rotation de la centrifugeuse 170. La séparation s'effectue en fonction des densités et des dimensions différentes des divers composants du liquide. Le composant du sang désiré est transféré dans le conteneur 148, au moyen de la pompe 200, par le tube compressible 146 qui relie la centrifugeuse au conteneur 148.

Lorsque la séparation est achevée, il est souvent souhaitable de déterminer la concentration du composant recueilli dans le conteneur 148. Afin que l'échantillon prélevé soit représentatif du liquide contenu dans le conteneur 148, un clamp 178 du type décrit plus haut peut être disposé sur la section du tube 146 s'étendant entre la centrifugeuse 170 et le conteneur 148, tel que cela est illustré sur la Figure 7. Le tube 146 est initialement disposé dans la première extrémité 182 de l'ouverture 180, laquelle est dimensionnée de façon à ne pas obturer le tube 146, et donc à ne pas empêcher l'écoulement du composant du sang récolté 176 en direction du conteneur 148. Lorsque la séparation est terminée, le tube 146 est alors généralement scellé et coupé afin de séparer le conteneur 148 de la centrifugeuse 170. Avant de prélever un échantillon du composant du sang récolté 176, le clamp 178 est mise en place sur le tube 146 en position d'occlusion à proximité de l'extrémité scellée afin de pouvoir transférer dans le conteneur 148 la plus grande quantité possible de composant du sang présent dans le tube 146.

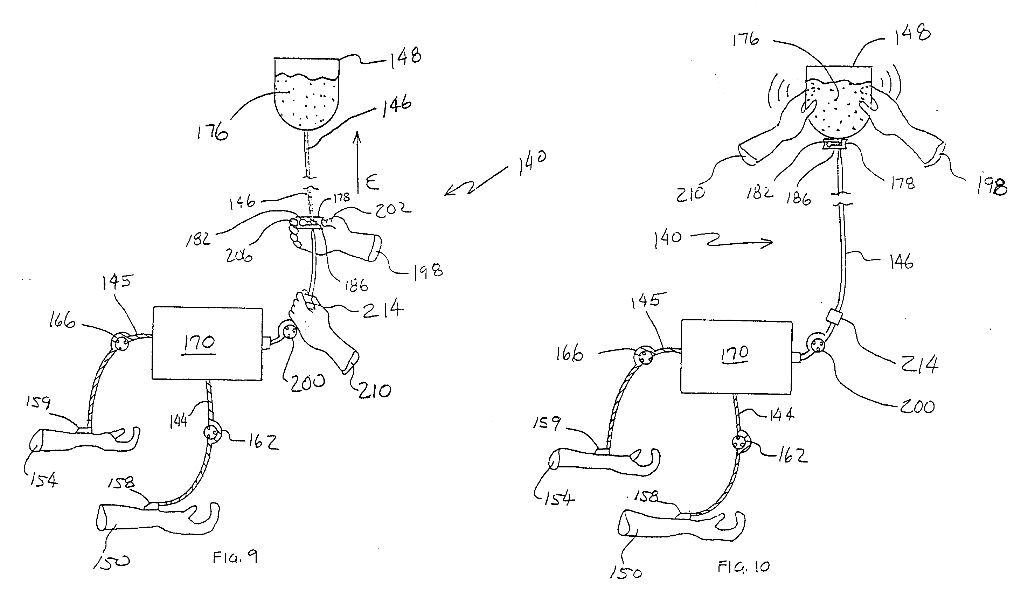

Pour provoquer l'occlusion du tube 146 par le clamp 178, l'utilisateur manipule le clamp 178 de façon à déplacer le tube 146 de la première extrémité 182 de l'ouverture 180 en direction de la seconde extrémité 186, laquelle est dimensionnée pour obturer le tube 146 par compression. Par exemple, l'utilisateur peut saisir le clamp 178 dans une main 198 en prenant les première et seconde surfaces de préhension 190, 194 entre le pouce 202 et l'index 206, et mouvoir ensuite le clamp 178 vis-à-vis du tube 146, transversalement à celui-ci, dans la direction de la flèche D représentée sur la Figure 8, jusqu'à ce que le tube 146 soit engagé dans la seconde extrémité 186. Pour cette manipulation, l'autre main 210 de l'utilisateur peut saisir toute partie adéquate du système 140 facilitant la mise en place du clamp 178, telle que la partie du tube 146 adjacente au clamp 178. On notera qu'une fois que le clamp 178 est dans cette position, il n'est pas nécessaire d'appliquer de force supplémentaire sur le clamp 178 pour maintenir l'occlusion du tube 146 (c'est-à-dire que l'obturation est maintenue par la simple interaction du tube 146 et de la seconde extrémité 186 de l'ouverture du clamp 178).

Une fois que le clamp 178 a été mis en place sur le tube de façon à l'obturer comme cela vient d'être décrit, l'utilisateur fait glisser le clamp 178 le long du tube 146 en direction du conteneur 148 dans le sens de la flèche E, tel que représenté sur la Figure 9. Ce mouvement de glissement du clamp 178 produit le déplacement d'un point de compression sur le tube 146 et provoque ainsi le transfert, dans le conteneur 148, du composant du sang 176 récolté qui se trouve dans le tube 146 entre le clamp 178 et le conteneur 148, le volume du composant contenu dans le conteneur augmentant d'autant. L'autre main 210 de l'utilisateur peut tenir la partie du tube 146 située en aval du clamp 178 lorsque le clamp 176 est déplacé le long du tube en direction du conteneur 148. Cependant, afin de fournir àl'utilisateur un point de préhension commode, un collier sensiblement incompressible 214 peut être assujetti au tube 146, comme cela est illustré sur les Figures 7 à 11. Le clamp 178 est glissé sur le tube 146 jusqu'à jouxter le conteneur 148 comme cela est illustré sur la Figure 10 de sorte que le maximum de composant du sang 176 présent dans le tube 146 est transféré dans le conteneur 148. Comme cela a été noté plus haut, il n'est pas nécessaire que l'utilisateur applique aucune force sur le clamp 178 pour maintenir l'occlusion du tube. De la sorte, comme cela est représenté sur la Figure 10, l'utilisateur a ses deux mains 198, 210 libres pour remuer le conteneur 148 et, ce faisant, en homogénéiser le contenu.

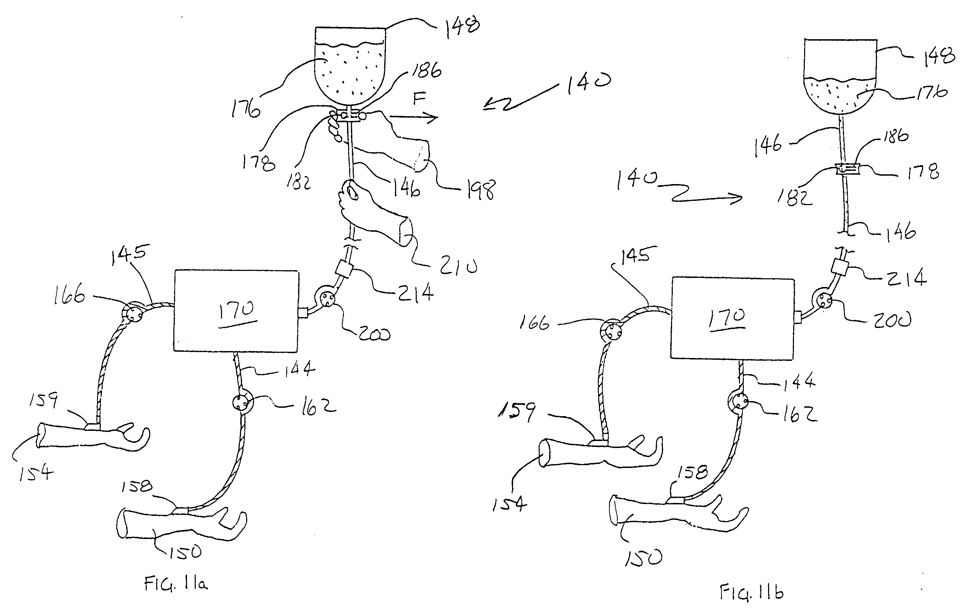

Une fois que le mélange a été effectué, l'utilisateur saisit le clamp 178, par exemple en prenant la première et la seconde surface de préhension 190, 194, entre le pouce 202 et l'index 206 d'une main 198. L'utilisateur exerce alors sur le clamp 178 une force dans la direction de la flèche F représentée sur la Figure 11A, pour rétablir le passage dans le tube 146 en disposant le tube 146 dans la première extrémité 182 de l'ouverture 180. Grâce au vide partiel créé en aval du clamp 178 lors du glissement du clamp 178 le long du tube scellé 146, le composant du sang 176 contenu dans le conteneur 148 s'écoule alors dans le tube 146 en direction de la partie scellée de celui-ci, comme cela est illustré sur la Figure 11B. Un échantillon du composant peut alors être prélevé dans le tube 146, par exemple en scellant et en détachant une partie du tube 146.

Compte tenu de ce qui vient d'être décrit, les spécialistes comprendront que l'application de l'invention à la séparation des composants du sang présente de nombreux avantages. Par exemple, une fois que le clamp 178 est engagé sur le tube 146 en position d'occlusion, il n'est pas nécessaire d'exercer aucune force supplémentaire sur le clamp 178 pour maintenir l'occlusion nécessaire à la purge du tube. En conséquence, l'utilisateur peut lâcher le clamp 178 à tout moment après qu'il a été mise en place sur le tube pour, le cas échéant, s'occuper du patient ou donneur, sans avoir à répéter les étapes du processus qui ont déjà été exécutées. Cela est d'un intérêt particulier lors du mélange du composant du sang 176 récolté dans le conteneur 148 car l'utilisateur peut utiliser ses deux mains dans cette étape du processus. Le clamp 178 est un élément bon marché et il peut être fabriqué facilement, par exemple par un procédé de moulage par injection.

La description de l'invention qui précède a été faite dans un but illustratif. Cette description ne doit pas être comprise comme limitant l'invention aux modes de réalisation représentés. Cette description a pour but d'expliquer la meilleure manière connue de mettre en oeuvre l'invention et de permettre au spécialiste d'utiliser l'invention telle que décrite ou d'autres modes de réalisation assortis des modifications nécessaires pour des applications particulières de l'invention, en particulier pour des applications dans les laboratoires de traitement du sang.

## Revendications

1. Dispositif de prise d'échantillon de liquide corporel (84) comprenant un tube (72) muni de moyens de prise d'échantillon (88), le tube (72) étant sensiblement compressible sur au moins une partie de sa longueur et ayant une première extrémité connectable à un conteneur (64) pour un liquide médical (68),
caractérisé en ce qu'il comprend des moyens de compression (96) montés sur le tube (72) pour coopérer avec le tube (72) dans une première position où le passage est libre à l'intérieur du tube (72) et dans une seconde position où le tube (72) est obturé par un point de compression, les moyens de compression (96) pouvant être déplacés par glissement le long du tube (72) dans la seconde position, et le glissement des moyens de compression (96) provoquant un déplacement de liquide dans le tube (72) en direction et/ou à partir du conteneur (64).

2. Dispositif selon la revendication 1, caractérisé en ce qu'il comporte des moyens de connexion intraveineux (80) connectés à une seconde extrémité du tube (72).

3. Dispositif selon une des revendications 1 ou 2, caractérisé en ce qu'il comporte des moyens de préhension (92) sensiblement incompressibles assujettis au tube (72) pour permettre la saisie du tube (72) sans qu'il en résulte une occlusion de celui-ci.

4. Dispositif selon une des revendications 1 à 3, caractérisé en ce que les moyens de prélèvement d'échantillon comprennent un site d'accès en ligne (88).

5. Dispositif selon une des revendications 1 à 4, caractérisé en ce que les moyens de compression (96) peuvent être déplacés par glissement le long du tube (72) dans la première position.

6. Dispositif selon une des revendications 1 à 5, caractérisé en ce que les moyens de compression (96) interagissent avec le tube (72) dans la seconde position de telle sorte que l'occlusion du tube (72) est maintenue sans qu'il soit nécessaire d'exercer une force extérieure sur les moyens de compression.

7. Dispositif selon une des revendications 1 à 6, caractérisé en ce que les moyens de compression (96) comprennent une plaque munie d'une ouverture (98) pour le passage du tube (72) ayant une première et une seconde extrémités (100, 104), la première extrémité (100) étant dimensionnée pour laisser le passage libre à l'intérieur du tube (72) et la seconde extrémité (104) étant dimensionnée pour exercer un point de compression sur le tube (72) résultant en l'obturation du tube (72).

8. Dispositif selon une des revendications 1 à 7, caractérisé en ce que le tube (72) est fait de chlorure de polyvinyle (PVC) et les moyens de compression (96) comprennent une partie en interaction avec le tube (72) faite de polyéthylène haute densité (HDPE).

9. Dispositif selon une des revendications 1 à 8, caractérisé en ce que le tube (72) est transparent.

10. Dispositif selon une des revendications 1 à 9, caractérisé en ce que le liquide médical est une solution de perfusion (68) et le liquide corporel est du sang (84).

11. Procédé pour prélever un échantillon de liquide corporel (84) à partir d'un tube (72), muni de moyens de prise d'échantillon (88), interconnectant un patient (76) a un conteneur (64) pour liquide médical (68), le tube (72) étant sensiblement compressible sur au moins une partie de sa longueur, caractérisé en ce qu'il comprend les étapes de :
- comprimer le tube (72) entre les moyens de prise d'échantillon (88) et le conteneur (64) de façon à obturer le tube (72) par un point de compression ;
- déplacer le point de compression le long du tube (72) en direction du conteneur (64) pour provoquer d'une part le transfert dans le conteneur (64) d'au moins une fraction du liquide (68) contenu dans le tube (72) et, d'autre part, l'introduction de liquide corporel (84) dans le tube (72) jusque au moins au niveau des moyens de prise d'échantillon (88) ;
- maintenir l'occlusion du tube (72) ; et
- prélever un échantillon de liquide corporel (84) par les moyens de prise d'échantillon (88).

12. Procédé selon la revendication 11, caractérisé en ce que le tube (72) est comprimé initialement en un point situé à une distance à partir du conteneur (64) supérieure ou égale à la distance séparant les moyens de prise d'échantillon (88) du patient (76).

13. Procédé selon la revendication 11 ou 12, caractérisé en ce que, après la prise d'échantillon, le passage dans le tube (72) est rétabli.
